# EUROPEAN PATENT APPLICATION

(11) **EP 2 481 444 A1**
(43) Date of publication of application: **01.08.2012**
(21) Application number: 10818721.2
(22) Date of filing: 14.09.2010
(51) Int. Cl.: A61M 37/00

(54) **DEVICE FOR INJECTING THERAPEUTIC SOLUTION**

(30) Priority: 22.09.2009 US 244586 P; 19.01.2010 JP 2010009173
(71) Applicant: Olympus Corporation, Shibuya-ku Tokyo 151-0072 (JP)
(72) Inventor: MIYOSHI, Shunichiro, Tokyo 151-0053 (JP); OKAZAKI, Yoshiro, Tokyo 151-0072 (JP); SUGAHARA, Michihiro, Tokyo 151-0072 (JP)
(74) Representative: Gunzelmann, Rainer
(86) International application number: PCT/JP2010/065851
(87) International publication number: WO 2011/037046

(57) **Abstract**

Therapeutic solution of an amount corresponding to a plurality of injections is injected with the distal end kept inserted in the pericardial cavity via an access port formed to penetrate under the xiphoid process. Provided is a therapeutic-solution injecting device (1) including an injection needle (3) for puncturing living tissue; a tube (6) having the injection needle (3) at a distal end thereof; a syringe connector (5) provided at a proximal end of the tube (6) and allowing connection and disconnection of a therapeutic-solution syringe (4) containing therapeutic solution; and a three-way valve (8) provided in the tube (6) between the syringe connector (5) and the injection needle (3) and having an open end.

## Description

### {Technical Field}

The present invention relates to therapeutic-solution injecting devices.

### {Background Art}

Conventionally, there has been a demand for realization of cell treatment for injecting stem cells in the form of a therapeutic solution into a lesion area to treat a heart disease, such as myocardial infarction.
As a device for injecting medicine into eyeball tissue, there is a known device that changes the entry angle for puncturing tissue by projecting and withdrawing a needle that is movable inside a rigid shaft in an axial direction via the distal end of the rigid shaft (e.g., see PTL 1).

### {Citation List}

### {Patent Literature}

{PTL 1} Japanese Translation of PCT International Application, Publication No. 2002-522116

### {Summary of Invention}

### {Technical Problem}

However, as opposed to the device in PTL 1 whose main application is injection of medicine into eyeball tissue, with a therapeutic-solution injecting device for cell treatment of heart disease, it is necessary to inject cells a plurality of times into different sites in the heart. Cells are injected into the heart by inserting the distal end of the therapeutic-solution injecting device into the pericardial cavity via an access port formed to penetrate under the xiphoid process. At this time, the therapeutic-solution injecting device must be inserted and withdrawn via the access port each time cells are injected, therefore there are the problems that the operation duration becomes longer and the burden on a patient is heavy.

The present invention has been made in view of the situation described above, and it is an object thereof to provide a therapeutic-solution injecting device with which it is possible to inject therapeutic solution of an amount corresponding to a plurality of injections with its distal end kept inserted in the pericardial cavity via an access port formed to penetrate under the xiphoid process.

### {Solution to Problem}

To achieve the above-described object, the present invention provides the following solutions.
A first aspect of the present invention is a therapeutic-solution injecting device including an injection needle for puncturing living tissue; a tube having the injection needle at a distal end thereof; a syringe connector provided at a proximal end of the tube and allowing connection and disconnection of a therapeutic-solution syringe containing therapeutic solution; and a three-way valve provided in the tube between the syringe connector and the injection needle and having an open end.

According to the first aspect of the present invention, the tube is inserted into the body via an access port formed penetrating the skin of the patient, and internal tissue is punctured with the injection needle provided at the distal end of the tube. Then, the therapeutic-solution syringe containing therapeutic solution is connected to the syringe connector provided at the proximal end of the tube, and the therapeutic solution is pushed out into the tube with the therapeutic-solution syringe. By the above procedure, it is possible to inject the therapeutic solution into the internal tissue via the tube.

In this case, when connecting the therapeutic-solution syringe to the syringe connector, air between the therapeutic-solution syringe and the syringe connector could be accidentally introduced into the therapeutic-solution syringe. The accidentally introduced air remains in the therapeutic solution as a bubble. Thus, by pushing out the therapeutic solution from the therapeutic-solution syringe with the three-way valve provided in the tube between the syringe connector and the injection needle to the open-end side, it is possible to discharge the accidentally introduced bubble to the outside from the open end of the three-way valve.

That is, it is possible to perform injection by attaching a new therapeutic-solution syringe to the syringe connector in place of a previous one each time therapeutic solution is injected and to discharge an accidentally introduced bubble each time. Accordingly, in treatment with therapeutic solution in which therapeutic solution is injected a plurality of times while changing the injection point, it is possible to supply additional therapeutic solution with the distal end of the tube kept inserted in the body. This alleviates the burden on the patient.

In the first aspect above, a medium syringe containing a medium may be connected at the proximal end of the tube, and a valve may be provided between the medium syringe and the syringe connector.
In this case, the valve is opened and the medium is supplied from the medium syringe to fill the tube with the medium, and then the valve is closed and the therapeutic-solution syringe connected to the syringe connector is pressed. At this time, a bubble accidentally introduced when connecting the therapeutic-solution syringe to the syringe connector is pushed out into the medium. In this state, by setting the three-way valve to the open-end side to open the valve and pressing the medium syringe, it is possible to discharge the bubble pushed out into the medium to the outside from the open end of the three-way valve with the medium.

In the first aspect above, a vibrator for vibrating the tube may be provided on the tube on the proximal-end side with respect to the syringe connector.
In this case, vibrating the tube with the vibrator, a bubble adhering to the inner wall of the tube can be easily moved. Accordingly, it is possible to discharge a bubble inside the tube more reliably to the outside from the open end of the three-way valve.

A second aspect of the present invention is a therapeutic-solution injecting device including an injection needle for puncturing living tissue; a tube having the injection needle at a distal end thereof; a syringe connector provided at a proximal end of the tube and allowing connection and disconnection of a therapeutic-solution syringe containing therapeutic solution; and a recess provided in the tube between the syringe connector and the injection needle and recessed upward on the inner surface of the tube.

According to the second aspect of the present invention, by attaching a new therapeutic-solution syringe to the syringe connector in place of a previous one, it is possible to arbitrarily change the amount of therapeutic solution to be injected into living tissue with the distal end of the tube kept inserted in the body. In this case, when connecting the therapeutic-solution syringe to the syringe connector, air could be accidentally introduced between the therapeutic-solution syringe and the syringe connector. When the therapeutic solution is pushed out with the therapeutic-solution syringe in this state, the accidentally introduced air advances into the tube. In the course of this advance, the air is trapped in the recess recessed upward, provided on the inner surface of the tube. Accordingly, it is possible to prevent the air accidentally introduced into the tube from reaching the injection needle at the distal end of the tube.

In the first and second aspects above, a bubble detecting unit that detects a bubble flowing through the tube, and an indicator that indicates detection of the bubble when the bubble is detected inside the tube by the bubble detecting unit may be provided.
In this case, if accidentally introduced air becomes a bubble and flows through the tube when injecting the therapeutic solution from the therapeutic-solution syringe, the bubble detecting unit detects the bubble, and the indicator gives an external indication of the detection. Accordingly, an operator can immediately stop injection of the therapeutic solution, so that it is possible to prevent injection of the bubble into living tissue more reliably.

In the first and second aspects above, a therapeutic-solution detecting unit that detects therapeutic solution flowing through the tube, and an indicator that indicates detection of the therapeutic solution when the therapeutic solution is detected inside the tube by the therapeutic-solution detecting unit may be provided.
In this case, when injecting the therapeutic solution from the therapeutic-solution syringe, an operator can readily recognize that the therapeutic solution has reached the position of the therapeutic-solution detecting unit.

In the first and second aspects above, an irradiating unit that irradiates the inside of the tube with light, a photodetector that detects diffused light diffused inside the tube or transmitted light transmitted through the tube, and a calculating unit that calculates the concentration of the therapeutic solution flowing through the tube based on the intensity of the diffused light or transmitted light detected by the photodetector may be provided.
In this case, for example, in a case where the concentration of therapeutic solution used varies, it is possible to suitably control the amount of therapeutic solution to be injected based on the concentration of therapeutic solution calculated by the calculating unit.

In the first and second aspects above, preferably, the tube has an inner diameter greater than or equal to 0.1 mm and less than or equal to 1 mm.
In this case, since a bubble accidentally introduced into the tube has a diameter sufficiently greater than the inner diameter of the tube, a gap that allows therapeutic solution to flow is not formed between the bubble and the inner wall of the tube. This prevents a problem where the therapeutic solution flows around the bubble and the bubble remains adhering to the inner wall of the tube when injecting therapeutic solution from the therapeutic-solution syringe, so that it is possible to push out the bubble reliably.

In the first and second aspects above, preferably, the volume of the tube from the syringe connector to the open end of the three-way valve is greater than or equal to the volume of the therapeutic-solution syringe.
In this case, when therapeutic solution is injected into the tube with the therapeutic-solution syringe, all of the therapeutic solution is stored in the tube. Accordingly, there is no need to switch the three-way valve in the course of injection in order to prevent overflow of the therapeutic solution from the open end, making it possible to inject all of the therapeutic solution with a single operation.

In the first and second aspects above, preferably, a volume changing mechanism that is provided at an intermediate point of the tube and that allows changing of the volume of the tube from the syringe connector to the open end of the three-way valve to a volume greater than or equal to the volume of the therapeutic-solution syringe is provided.
In this case, by injecting therapeutic solution into the tube with the volume of the tube increased by the volume changing mechanism, it is possible to inject all of the therapeutic solution with a single operation.

### {Advantageous Effects of Invention}

According to the first and second aspects of the present invention, an advantage is afforded in that it is possible to inject therapeutic solution of an amount corresponding to a plurality of injections with the distal end kept inserted in the pericardial cavity via an access port formed to penetrate under the xiphoid process.

### {Brief Description of Drawings}

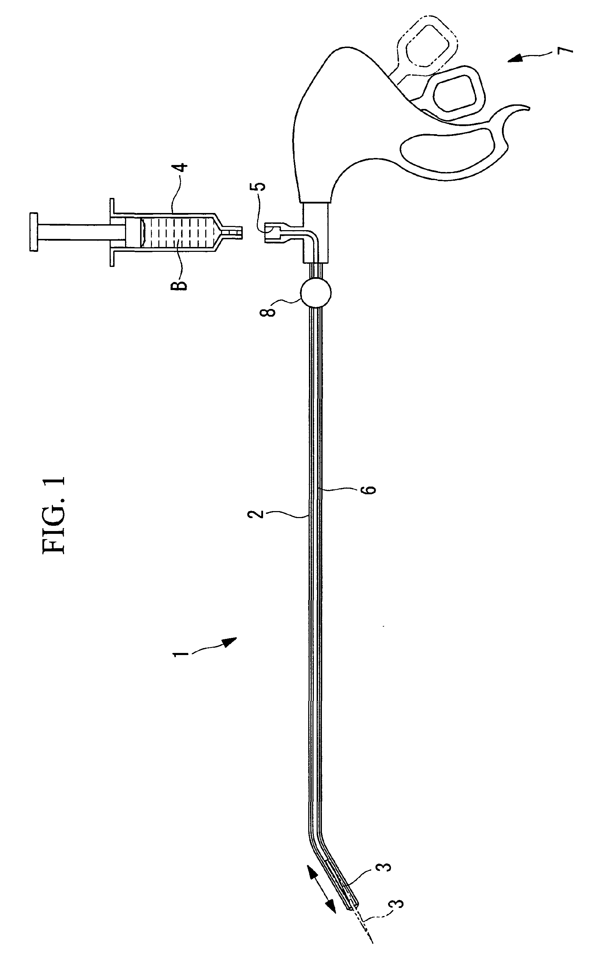

{Fig. 1}
   Fig. 1 is a partially sectional, overall configuration diagram illustrating a therapeutic-solution injecting device according to an embodiment of the present invention;
{Fig. 2A}
   Fig. 2A is a partially cutaway, partial vertical section illustrating a first flow path of a three-way valve and flow of therapeutic solution or physiological saline in the therapeutic-solution injecting device in Fig. 1;
{Fig. 2B}
   Fig. 2B is a partially cutaway, partial vertical section illustrating a second flow path of the three-way valve and flow of therapeutic solution or physiological saline in the therapeutic-solution injecting device in Fig. 1;
{Fig. 3A}
   Fig. 3A is a partially cutaway, partial vertical section illustrating a flow path for supplying physiological saline to an injection needle in a modification of the therapeutic-solution injecting device in Fig. 1, in which two three-way valves are provided;
{Fig. 3B}
   Fig. 3B is a partially cutaway, partial vertical section illustrating a flow path for discharging a bubble in the modification of the therapeutic-solution injecting device in Fig. 1, in which two three-way valves are provided;
{Fig. 3C}
   Fig. 3C is a partially cutaway, partial vertical section illustrating a flow path for supplying therapeutic solution into a tube in the modification of the therapeutic-solution injecting device in Fig. 1, in which two three-way valves are provided;
{Fig. 4}
   Fig. 4 is a schematic diagram illustrating another modification of the therapeutic-solution injecting device in Fig. 1;
{Fig. 5}
   Fig. 5 is a partially cutaway, partial vertical section illustrating another modification of the therapeutic-solution injecting device in Fig. 1;
{Fig. 6}
   Fig. 6 is a schematic diagram illustrating another modification of the therapeutic-solution injecting device in Fig. 1;
{Fig. 7}
   Fig. 7 is a partially sectional, overall configuration diagram illustrating another modification of the therapeutic-solution injecting device in Fig. 1;
{Fig. 8}
   Fig. 8 is a partially sectional, overall configuration diagram illustrating another modification of the therapeutic-solution injecting device in Fig. 1;
{Fig. 9}
   Fig. 9 is a partial vertical section illustrating another modification of the therapeutic-solution injecting device in Fig. 1;
{Fig. 10A}
   Fig. 10A is an illustration of an example of a volume changing mechanism in Fig. 9, illustrating a configuration in a case where the volume is changed by pressing the tube;
{Fig. 10B}
   Fig. 10B is an illustration of the tube in a pressed state in the case of Fig. 10A;
{Fig. 11A}
   Fig. 11A is an illustration of another example of the volume changing mechanism in Fig. 9, illustrating another configuration in a case where the volume is changed by pressing the tube;
{Fig. 11B}
   Fig. 11B is an illustration of the tube in a pressed state in the case of Fig. 11A; and
{Fig. 12}
   Fig. 12 is a partial vertical section illustrating another example of the volume changing mechanism in Fig. 9, illustrating a configuration in a case where the volume of a flow path is changed by using a flow-path switching member.

### {Description of Embodiments}

A therapeutic-solution injecting device 1 according to an embodiment of the present invention will be described below with reference to the drawings.
As shown in Fig. 1, the therapeutic-solution injecting device 1 according to this embodiment includes a rigid shaft 2 whose distal end is inserted into the pericardial cavity via an access port formed penetrating epidermal tissue under the xiphoid process, a tube 6 accommodated inside the rigid shaft 2, having an injection needle 3 at its distal end, and having a syringe connector 5 for connecting a therapeutic-solution syringe 4 at its proximal end, a needle driving handle 7 for moving the injection needle 3 provided at the distal end of the tube 6 in a lengthwise direction thereof so that the injection needle 3 is projected and withdrawn via the distal end of the rigid shaft 2, and a three-way valve 8 provided in the tube 6.

The rigid shaft 2 and the tube 6 are formed of a transparent material so that therapeutic solution B including stem cells and flowing through the tube 6 can be seen from the outside. The syringe connector 5 is a port that allows attachment and detachment of a syringe.

As the tube 6, a tube having an inner diameter greater than or equal to 0.1 mm and less than or equal to 1 mm at least in the region from the syringe connector 5 to the three-way valve 8 is used. An inner diameter of the tube 6 of less than 0.1 mm is undesirable since a high pressure is required to inject the therapeutic solution B with the therapeutic-solution syringe 4. On the other hand, with an inner diameter of the tube 6 of greater than 1 mm, the diameter of a bubble introduced into the tube 6 could be less than the inner diameter of the tube 6. This is undesirable since, in this case, the therapeutic solution B might pass through a gap formed between the bubble and the inner wall of the tube 6 and go beyond the position of the bubble, failing to push out the bubble with the therapeutic solution B.

The needle driving handle 7 is configured so that the injection needle 3 is projected from the distal end of the rigid shaft 2 when it is moved in an extending direction (moved to the position indicated by a chain line in the figure) and so that the injection needle 3 is withdrawn inside the rigid shaft 2 when it is moved in a retracting direction (moved to the position indicated by a solid line in the figure). Furthermore, the needle driving handle 7 is urged in the retracting direction by a spring (not shown). Thus, unless the needle driving handle 7 is gripped, the injection needle 3 is accommodated inside the rigid shaft 2.

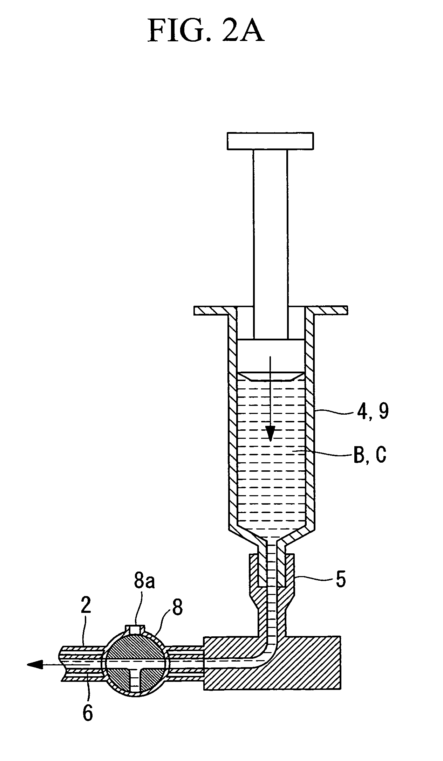

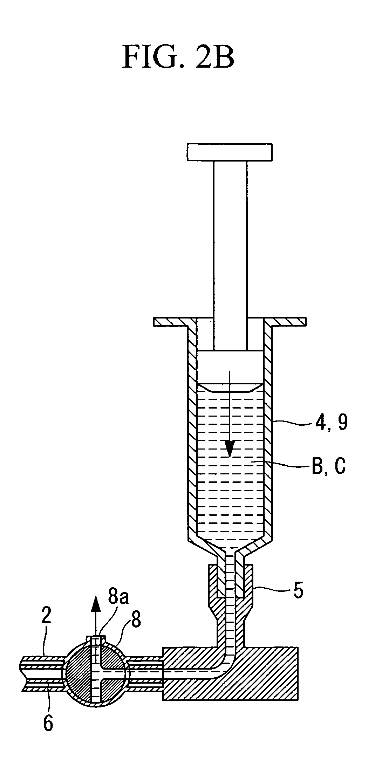

The three-way valve 8 is provided at an intermediate point of the tube 6 between the injection needle 3 and the syringe connector 5. The three-way valve 8 has a first flow path forming a flow path inside the tube 6 from the syringe connector 5 to the injection needle 3, as shown in Fig. 2A, and a second flow path with which the flow path from the syringe connector 5 is opened to the outside, as shown in Fig. 2B.

It is possible to discharge the therapeutic solution B contained in the therapeutic-solution syringe 4 from the injection needle 3 via the tube 6 by operating the three-way valve 8 to select the first flow path. On the other hand, it is possible to discharge a bubble included in the therapeutic solution B discharged from the syringe connector 5 to the outside from an open end 8a of the three-way valve 8 by operating the three-way valve 8 to select the second flow path.

The operation of the thus-configured therapeutic-solution injecting device 1 according to this embodiment will be described below.
In order to inject the therapeutic solution B including stem cells into a lesion area associated with a heart disease by using the therapeutic-solution injecting device 1 according to this embodiment, first, a medium syringe 9 containing physiological saline (medium) C is connected to the syringe connector 5. Then, with the three-way valve 8 switched to the first flow path, the medium syringe 9 is operated to fill the entire flow path inside the tube 6 with the physiological saline.

Then, the medium syringe 9 at the syringe connector 5 is detached, and the therapeutic-solution syringe 4 is connected. By detaching the medium syringe 9, the syringe connector 5 is opened to the air, and by attaching the therapeutic-solution syringe 4, the syringe connector 5 is closed again. At this time, in some cases, a bubble may enter between the distal end of the therapeutic-solution syringe 4 and the syringe connector 5, whereby the bubble is accidentally introduced into the flow path closed with the attached therapeutic-solution syringe 4.

Then, as shown in Fig. 2B, with the three-way valve 8 switched to the second flow path, the therapeutic-solution syringe 4 is operated to push out some of the therapeutic solution B from the syringe connector 5 into the tube 6. The air accidentally introduced into the flow path when connecting the therapeutic-solution syringe 4 is pushed out into the tube 6 with the therapeutic solution B. At this time, since the second flow path is opened to the air at the open end 8a of the three-way valve 8 by switching of the three-way valve 8, the bubble pushed out into the tube 6 is discharged to the outside from the open end 8a of the three-way valve 8.

In this state, as shown in Fig. 2A, the three-way valve 8 is again switched to the first flow path. Thus, no bubble is accidentally introduced into the therapeutic solution B that is subsequently pushed out by operating the therapeutic-solution syringe 4. Therefore, it is possible to prevent occurrence of a problem where a bubble is injected into the heart lesion area.
Furthermore, in this state, since the therapeutic solution B remains in the tube 6, the therapeutic-solution syringe 4 is detached from the syringe connector 5, and instead the medium syringe 9 is attached to the syringe connector 5. Air could be accidentally introduced also at this time. Therefore, similarly, the three-way valve 8 is switched to the second flow path, and the medium syringe 9 is operated to discharge the accidentally introduced bubble to the outside from the open end 8a of the three-way valve 8.

Then, after the bubble is discharged, the three-way valve 8 is switched to the first flow path, and the medium syringe 9 is operated to push out the physiological saline C. Thus, it is possible to inject the therapeutic solution B remaining in the tube 6 into the heart lesion area from the injection needle 3.
By repeating the above operation at the time when the physiological saline C has been injected to such an extent that the entire tube 6 is filled therewith, it is possible to perform injection a plurality of times while adding the therapeutic solution B. An advantage is afforded in that, at the time of this operation, it is possible to prevent occurrence of a problem where a bubble is injected into the lesion area.

As described above, with the therapeutic-solution injecting device 1 according to this embodiment, it is possible to attach and detach the therapeutic-solution syringe 4 to and from the syringe connector 5. Furthermore, even if air is accidentally introduced by such attachment and detachment, it is possible to discharge the air to the outside. Thus, for example, by attaching a new therapeutic-solution syringe 4 containing therapeutic solution B for one shot to the syringe connector 5 in place of a previous one, it is possible to perform cell injection an arbitrary number of times as needed with the distal end of the rigid shaft 2 kept inserted in the pericardial cavity via the access port.
Accordingly, an advantage is afforded in that an operation is prevented from taking a long time due to a plurality of insertions and removals of the rigid shaft 2, alleviating the burden on the patient.

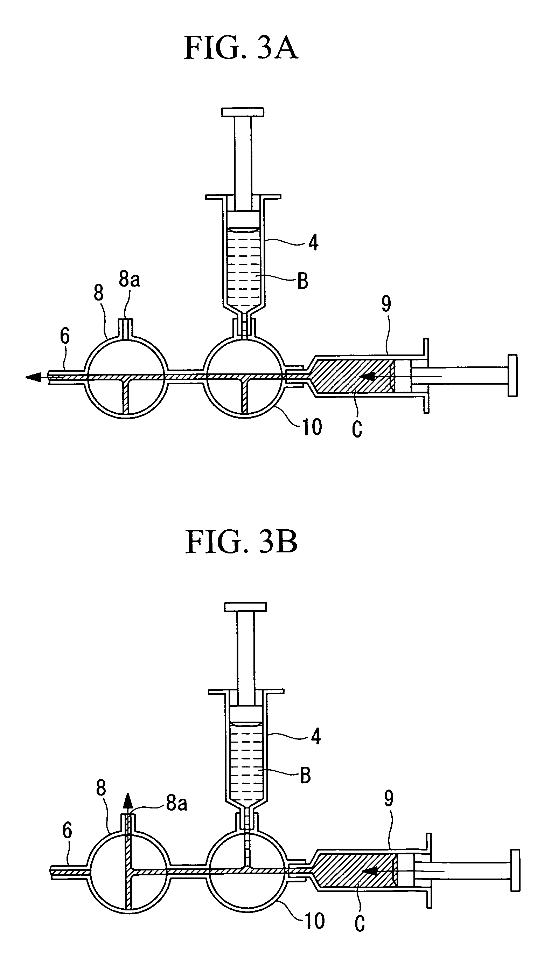

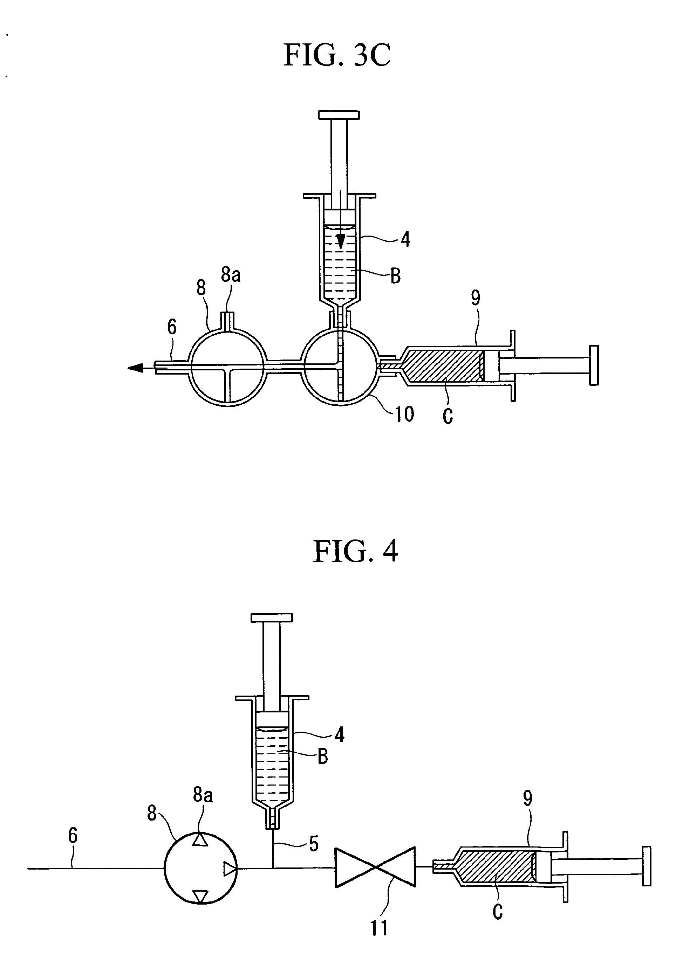

Although the therapeutic-solution syringe 4 and the medium syringe 9 are attached to the single syringe connector 5 by switching in this embodiment, alternatively, as shown in Fig. 3, by using another three-way valve 10, the therapeutic-solution syringe 4 and the medium syringe 9 may both be attached in such a manner that either one can be selected. Also in this case, the therapeutic solution B in the therapeutic-solution syringe 4 is completely used on one or more injections, so that it is necessary to attach a new therapeutic-solution syringe 4 to the syringe connector 5 in place of a previous one.

That is, first, as shown in Fig. 3A, the three-way valves 8 and 10 are operated to form a flow path such that only the medium syringe 9 is connected to the injection needle 3 via the tube 6. Then, with the tube 6 filled with the physiological saline C contained in the medium syringe 9, the distal end of the rigid shaft 2 is inserted into the pericardial cavity.
Then, as shown in Fig. 3C, the flow path is switched to the therapeutic-solution syringe 4, and a certain amount of the therapeutic solution B is pushed out to discharge the physiological saline C inside the tube 6 from the distal end of the injection needle 3, whereby the tube 6 is filled with the therapeutic solution B.

In this state, the heart lesion area is punctured with the injection needle 3, and the three-way valve 10 is operated as shown in Fig. 3A to ensure a flow path connected from the medium syringe 9 to the injection needle 3, whereby the physiological saline C is supplied to the tube 6. Thus, the therapeutic solution B filling the tube 6 is discharged from the injection needle 3 and injected into the lesion area.

When the therapeutic solution B filling the tube 6 has been fully discharged, the therapeutic-solution syringe 4 is detached from the syringe connector 5, and a new therapeutic-solution syringe 4 filled with therapeutic solution B is attached instead. At the same time, as shown in Fig. 3B, the three-way valves 8 and 10 are operated so that the therapeutic-solution syringe 4 and the medium syringe 9 are both connected to the open end 8a of the three-way valve 8.

In this state, first, the therapeutic-solution syringe 4 is pressed to push out a bubble remaining at the interface between the therapeutic solution B and the physiological saline C into the three-way valve 10. Then, the medium syringe 9 is pressed to push out the bubble pushed out into the three-way valve 10 toward the open end 8a of the three-way valve 8 with the physiological saline C. Thus, the bubble is discharged from the open end 8a, so that the bubble is prevented from being injected into the heart lesion area. Furthermore, an advantage is afforded in that, when the bubble is discharged from the open end 8a, it suffices to discharge the physiological saline C from the open end 8a, avoiding waste of the valuable therapeutic solution B.

In this embodiment, as shown in Fig. 4, a valve 11 may be provided in the tube 6 between the syringe connector 5 and the medium syringe 9.
In this case, with the valve 11 closed, it is possible to push out a bubble from the therapeutic-solution syringe 4 into the syringe connector 5 with the therapeutic solution B, and then, with the valve 11 opened, it is possible to discharge the bubble from the open end 8a of the three-way valve 8 with the physiological saline C.

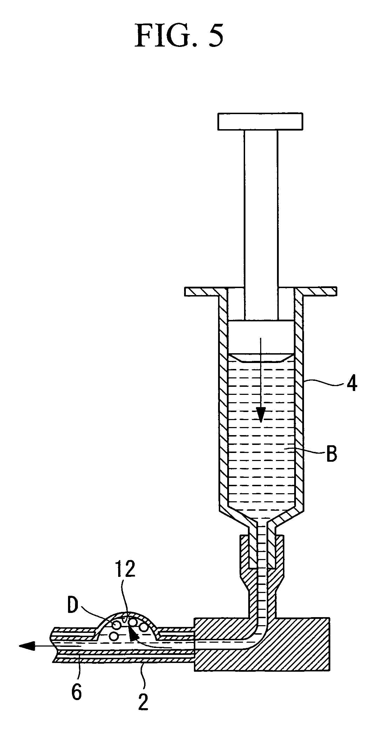

In this embodiment, instead of the three-way valve 8, as shown in Fig. 5, a recess 12 that is recessed upward may be provided at an intermediate point on the inner surface of the tube 6.
In this case, a bubble D accidentally introduced by changing the therapeutic-solution syringe 4 is captured at the recess 12 while flowing through the tube 6. Accordingly, even when the bubble D is accidentally introduced, the bubble D does not reach the injection needle 3, so that the bubble D is prevented from being injected into the lesion area.

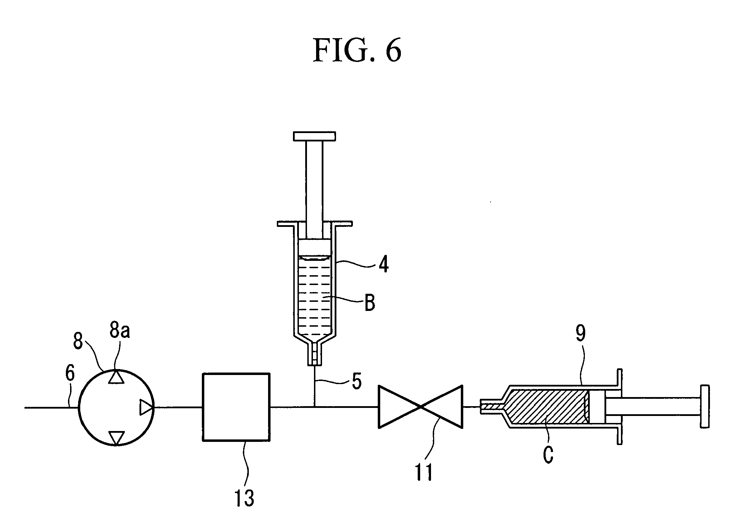

In this embodiment, as shown in Fig. 6, a vibrator 13 for vibrating the tube 6 may be provided at the upstream side of the three-way valve 8 or the recess 12. In this case, the tube 6 is vibrated by the operation of the vibrator 13. Accordingly, an advantage is afforded in that a bubble adhering to the inner wall of the tube 6 is easier to move with the therapeutic solution B or the physiological saline C, making it possible to readily discharge or capture the bubble.

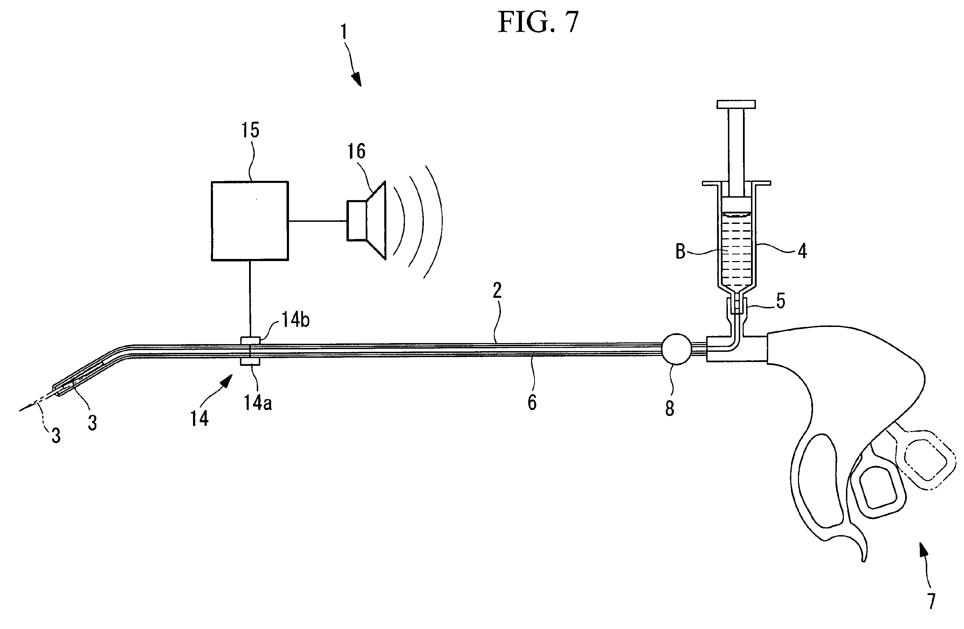

In this embodiment, as shown in Fig. 7, a sensor (bubble detecting unit) 14 having a light emitter 14a and a photoreceptor 14b disposed on either side of the tube 6 in a radial direction, a signal processor 15 that processes signals output from the sensor 14, and a speaker (indicator) 16 that converts the signals processed by the signal processor 15 into sound and outputs the sound may be provided.

In this case, a change in the transmittance of the therapeutic solution B or the physiological saline C flowing through the tube 6 is indicated in the form of sound from the speaker. Specifically, with the physiological saline C alone, the sound does not change significantly; with the therapeutic solution B, a sound different from that in the case of the physiological saline C is generated; when a bubble D is accidentally introduced, the situation is indicated in the form of continual changes in sound. Accordingly, an operator can recognize the state of the liquid being injected based on a change in sound. This makes it possible to perform an operation with the eyes kept on a monitor displaying an endoscopic image or on the patient.

In the case of a change from a state where the physiological saline C is detected to a state where the therapeutic solution B is detected, the operator can puncture the lesion area with the injection needle 3. In the opposite case, the operator can recognize that the injection needle 3 with which the lesion is punctured must be withdrawn. Furthermore, when a bubble D is detected inside the tube 6 during injection of the therapeutic solution B, the operator can stop injection of the therapeutic solution B immediately, avoiding injection of the bubble D into the lesion area.
Furthermore, instead of indicating the state of the liquid flowing through the tube 6 by means of sound, alternatively, indication may be given by means of light or a display on a monitor.

Furthermore, the sensor (therapeutic-solution detecting unit) 14 may detect the passage of the therapeutic solution B based on the intensity of light received by the photoreceptor 14b, i.e., the light transmittance inside the tube 6. When the therapeutic solution B is detected by the sensor 14, the indicator notifies the operator of the detection. For example, the indicator may be the speaker 16 or a lamp (not shown). Accordingly, the operator can readily recognize that the therapeutic solution B injected into the tube 6 at the proximal end has reached the position of the sensor 14.

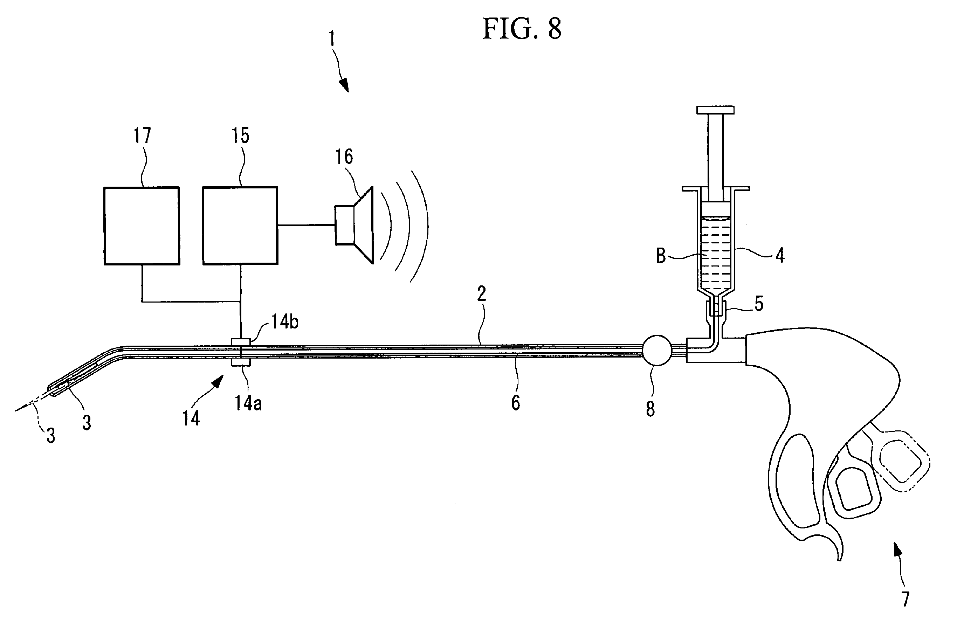

Furthermore, in this embodiment, a calculator 17 that calculates the concentration of the therapeutic solution B based on the intensity of light received by the photoreceptor (photodetector) 14b may be provided. Although the calculating unit 17 and the signal processor 15 use the common sensor 14 in Fig. 8, alternatively, the calculating unit 17 may use another sensor (not shown) separate from that for the signal processor 15. Furthermore, although the photoreceptor 14b is configured to receive transmitted light transmitted through the tube 6 in Fig. 8, alternatively, the photoreceptor 14b may be provided on the same side of the tube 6 as the light emitter (irradiating unit) 14a and configured to receive diffused light diffused inside the tube 6 and returned therefrom. The value of the concentration of the therapeutic solution B calculated by the calculating unit 17 is displayed on a monitor or the like (not shown).

Accordingly, for example, even in a case where the concentration of the therapeutic solution B varies depending on the operation, by monitoring the concentration of the therapeutic solution B and adjusting the amount of injected therapeutic solution B based on the concentration, it is possible to suitably control the amount of therapeutic solution B injected into a lesion area each time.

In this embodiment, the length of the tube 6 from the syringe connector 5 to the three-way valve 8 may be designed such that the volume of the tube 6 from the syringe connector 5 to the three-way valve 8 is greater than or equal to the volume of the therapeutic-solution syringe 4. In this case, even if all the therapeutic solution B contained in the therapeutic-solution syringe 4 is injected into the tube 6 at once, the therapeutic solution B does not overflow from the open end 8a of the three-way valve 8. Accordingly, it is possible to eliminate switching of the three-way valve 8 during injection of the therapeutic solution B, making it possible to inject all the therapeutic solution B with a single operation. Furthermore, there is no need to pay attention to the position of the distal-end surface of the therapeutic solution B during injection, so that operation is facilitated. Furthermore, it is possible to prevent the therapeutic solution B from overflowing from the open end 8a and being wasted or to prevent inaccuracy of the amount of the therapeutic solution B injected into a lesion area.

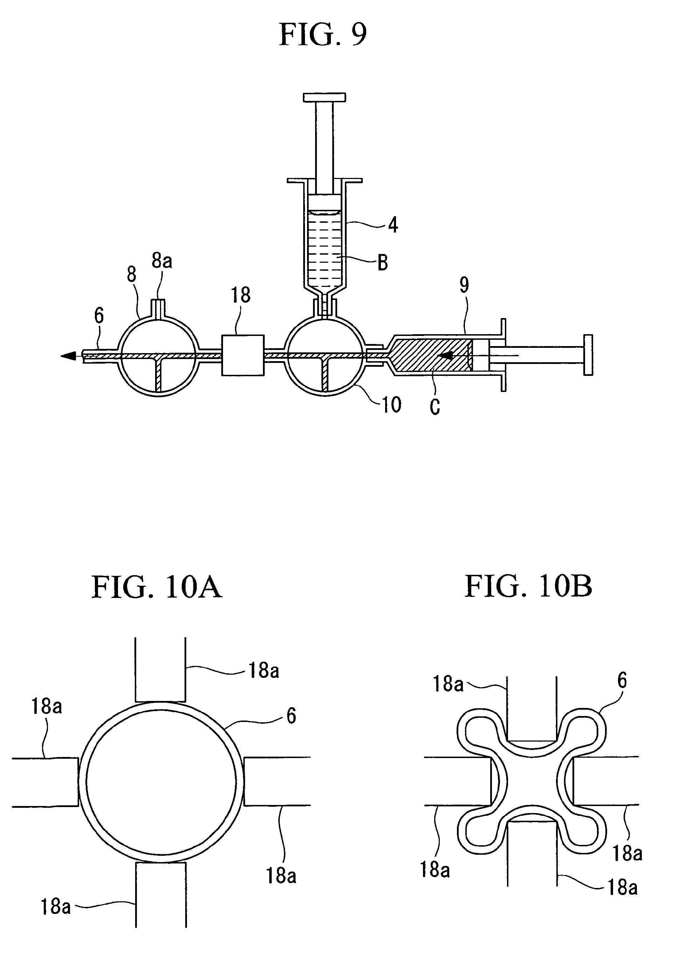

Alternatively, as shown in Fig. 9, a volume changing mechanism 18 for changing the volume of the tube 6 may be provided at an intermediate point of the tube 6 between the syringe connector 5 and the three-way valve 8. The volume changing mechanism 18 allows changing of the volume of the tube 6 from the syringe connector 5 to the three-way valve 8 to a volume greater than or equal to the volume of the therapeutic-solution syringe 4. In this case, by increasing the volume of the tube 6 only when needed, such as when injecting the therapeutic solution B, it is possible to reduce the amount of other medicine used, such as a medium. Furthermore, for example, even when therapeutic-solution syringes 4 of different capacities are used, it is possible to change the volume of the tube 6 each time in accordance with the amount of therapeutic solution B to be injected.

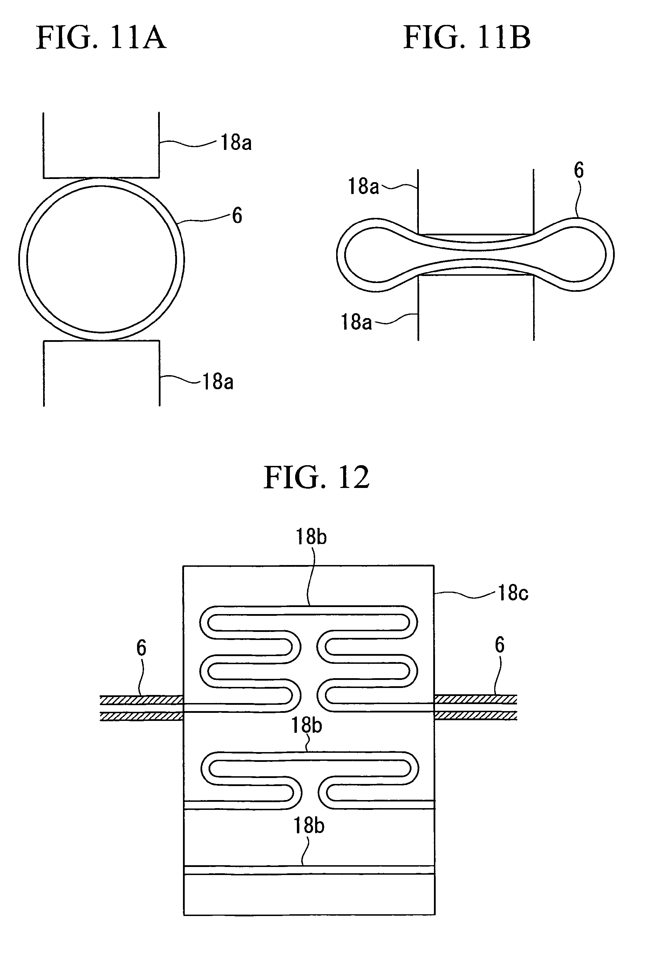

In order to implement the volume changing mechanism 18, for example, the tube 6 formed of an elastic material, such as a rubber, may be stretched in the lengthwise direction or pressed in a radial direction in the region between the syringe connector 5 and the three-way valve 8. For example, as shown in Figs. 10A or 11A, by providing one pair or two pairs of pressing members 18a on either side of the tube 6 in a radial direction and moving the pressing members 18a closer to each other with an actuator or the like (not shown), it is possible to press the tube 6, as shown in Fig. 10B or Fig. 11B. Alternatively, the volume changing mechanism 18 may be implemented by inflating or deflating a balloon (not shown) provided inside the tube 6. Alternatively, as shown in Fig. 12, the volume changing mechanism 18 may be implemented by providing a flow-path switching member 18c forming flow paths 18b having different capacities and changing the flow path 18b communicating with the tube 6.

### {Reference Signs List}

- B:: therapeutic solution
- D:: bubble
- 1:: therapeutic-solution injecting device
- 3:: injection needle
- 4:: therapeutic-solution syringe
- 5:: syringe connector
- 6:: tube
- 8:: three-way valve
- 8a:: open end
- 9:: medium syringe
- 11:: valve
- 12:: recess
- 13:: vibrator
- 14:: sensor
- 16:: speaker (indicator)
- 17:: calculating unit
- 18:: volume changing mechanism
- 18a:: pressing member
- 18b:: flow path
- 18c:: flow-path switching member

## Claims

1. A therapeutic-solution injecting device comprising:
an injection needle for puncturing living tissue;
a tube having the injection needle at a distal end thereof;
a syringe connector provided at a proximal end of the tube and allowing connection and disconnection of a therapeutic-solution syringe containing therapeutic solution; and
a three-way valve provided in the tube between the syringe connector and the injection needle and having an open end.

2. A therapeutic-solution injecting device according to Claim 1,
wherein a medium syringe containing a medium is connected at the proximal end of the tube, and
wherein a valve is provided between the medium syringe and the syringe connector.

3. A therapeutic-solution injecting device according to Claim 1 or 2, further comprising a vibrator for vibrating the tube, provided on the tube on the proximal-end side with respect to the syringe connector.

4. A therapeutic-solution injecting device comprising:
an injection needle for puncturing living tissue;
a tube having the injection needle at a distal end thereof;
a syringe connector provided at a proximal end of the tube and allowing connection and disconnection of a therapeutic-solution syringe containing therapeutic solution; and
a recess provided in the tube between the syringe connector and the injection needle and recessed upward on the inner surface of the tube.

5. A therapeutic-solution injecting device according to any one of Claims 1 to 4, further comprising:
a bubble detecting unit that detects a bubble flowing through the tube; and
an indicator that indicates detection of the bubble when the bubble is detected inside the tube by the bubble detecting unit.

6. A therapeutic-solution injecting device according to any one of Claims 1 to 5, further comprising:
a therapeutic-solution detecting unit that detects therapeutic solution flowing through the tube; and
an indicator that indicates detection of the therapeutic solution when the therapeutic solution is detected inside the tube by the therapeutic-solution detecting unit.

7. A therapeutic-solution injecting device according to any one of Claims 1 to 6, further comprising:
an irradiating unit that irradiates the inside of the tube with light;
a photodetector that detects diffused light diffused inside the tube or transmitted light transmitted through the tube; and
a calculating unit that calculates the concentration of the therapeutic solution flowing through the tube based on the intensity of the diffused light or transmitted light detected by the photodetector.

8. A therapeutic-solution injecting device according to any one of Claims 1 to 7, wherein the tube has an inner diameter greater than or equal to 0.1 mm and less than or equal to 1 mm.

9. A therapeutic-solution injecting device according to any one of Claims 1 to 8, wherein the volume of the tube from the syringe connector to the open end of the three-way valve is greater than or equal to the volume of the therapeutic-solution syringe.

10. A therapeutic-solution injecting device according to any one of Claims 1 to 8, further comprising a volume changing mechanism that is provided at an intermediate point of the tube and that allows changing the volume of the tube from the syringe connector to the open end of the three-way valve to a volume greater than or equal to the volume of the therapeutic-solution syringe.
